# EUROPEAN PATENT APPLICATION

(11) **EP 4 141 031 A1**
(43) Date of publication of application: **01.03.2023**
(21) Application number: 21793385.2
(22) Date of filing: 19.04.2021
(51) Int. Cl.: C07K 16/28, C12N 15/13, C12N 5/00, C12N 15/63, A61K 39/395, A61P 37/00, A61P 35/00, G01N 33/53

(54) **ANTIBODY BINDING WITH SPECIFIC EPITOPE IN HUMAN IL-4R ALPHA AND APPLICATIONS OF ANTIBODY**

(30) Priority: 24.04.2020 CN 202010331685
(71) Applicant: SUZHOU CONNECT BIOPHARMACEUTICALS, LTD., Taicang, Jiangsu 215400 (CN)
(72) Inventor: YANG, Xin, Taicang City, Jiangsu 215400 (CN); ZHANG, Limin, Taicang City, Jiangsu 215400 (CN); JIANG, Jie, Taicang City, Jiangsu 215400 (CN); XIA, Jing, Taicang City, Jiangsu 215400 (CN); PAN, Wubin, Taicang City, Jiangsu 215400 (CN); ZHENG, Wei, San Diego California 92130 (US)
(74) Representative: Schiweck Weinzierl Koch Patentanwälte Partnerschaft mbB
(86) International application number: PCT/CN2021/088139
(87) International publication number: WO 2021/213329

(57) **Abstract**

Disclosed is a human IL-4R antibody or an antigen binding fragment thereof. The antibody or the antigen binding fragment thereof specifically binds with human IL-4Rα and binds with a specific epitope in human IL-4Rα. Moreover, also provided in the present disclosure are uses of the epitope in screening a human IL-4R binding agent or blocking agent for a human IL-4/IL-13 signal transduction pathway or uses in assessing the efficacy of the binding agent or blocking agent by using the specific epitope.

## Description

### CROSS REFENECE OF RELATED APPLICATION(S)

The present application claims the priority benefit of Chinese Patent Application No. CN202010331685.3 filed on April 24, 2020, which is hereby incorporated by reference in its entirety.

### TECHNICAL FIELD

The present disclosure relates to the field of biological pharmacy, in particular to an antibody capable of binding to a specific epitope of human IL-4Rα and applications thereof.

### BACKGROUND OF THE INVENTION

Human interleukin 4 receptor (IL-4R) is a type I transmembrane protein which is widely expressed in various tissues and organs throughout the body, particularly in immune organs, such as tonsils, appendices, lymph nodes, spleen, and bone marrow. IL-4R, when binds to ligands such as interleukin 4 (IL-4) and interleukin 13 (IL-13), can exert a variety of immunomodulatory effects, for example, to promote differentiation of Th2 cells, regulate secretion of IgE antibodies by B cells, and stimulate macrophage alternative activation, and the like.

IL-4R is a heterodimer composed of two polypeptide chains, one of which, the α chain (IL-4Rα) has a high affinity for IL-4. Also, IL-4Rα forms another form of IL-4R heterodimer with the α chain of IL-13 cell surface receptor (IL-13Rα), and thus has a high affinity for IL-13 as well.

A soluble form (sIL-4Rα) of the protein can be produced from IL-4Rα. Such soluble form of the protein can inhibit a range of inflammation-associated signaling, for example, IL-4 mediated cell proliferation and T-cell mediated IL-5 upregulation and the like. Therefore, a blocking antibody targeted to the protein is beneficial to treating and relieving allergic rhinitis, nasosinusitis, asthma or eczema and other diseases. Also, both IL-4 and IL-13 are cytokines having a very broad spectrum of biological activities, involved in a wide variety of inflammation-related responses, most produced by activated T cells, monocytes, mast cells, basophils and eosinophils. The two interleukins have many commonalities in biological functions, and their main biological effects include stimulating effect on differentiation and proliferation of TH2 cells, stimulating effect on secretion of IgE antibodies by activated B cells, and the like. Researches show that IL-4 and IL-13 play a very important role in mediating the immune response of autoimmune diseases, allergic diseases, tumors and other diseases, and always are one of the research hotspots that people pay attention to.

Since sIL-4Rα dominates the binding to IL-4 and relates to other cytokines, currently many reseaches are on going towards antibodies targeted to sIL-4Rα, and it has been clinically demonstrated that human monoclonal antibodies directed against the target are effective in the alleviation and treatment of diseases such as asthma, atopic dermatitis, etc. However, epitopes of sIL-4Rα in immune responses are rarely studied, and specific epitopes are not clearly known yet.

### SUMMARY OF THE INVENTION

For the above technical problem, the present inventors have studied the epitopes of sIL-4Rα. According to the species specificities shown by newly obtained antibodies when they reacted with human sIL-4Rα and cynomolgus sIL-4Rα, the inventors selected some positions in sIL-4Rα and mutated the positions, performed relevant experiments, and found out several critical amino acids of binding epitopes. These results about epitope are of great importance for further studies on sIL-4Rα and related antibodies.

Accordingly, an object of the present disclosure to provide an antibody or antigen-binding fragment thereof that binds to human interleukin 4 receptor (IL-4R), the antibody or antigen-binding fragment thereof specifically binding to a specific epitope of the alpha chain of human IL-4R (IL-4Rα).

On the basis of the antibody or antigen-binding fragment thereof provided by the present disclosure, another object of the present disclosure is to provide a nucleic acid molecule comprising a nucleotide sequence encoding the key domain(s) in the antibody or antigen-binding fragment thereof according to the present disclosure; to provide a vector comprising the nucleic acid molecule; to provide a host cell containing the vector; to provide a method for producing the antibody or antigen-binding fragment thereof; to provide a pharmaceutical composition comprising the antibody or antigen-binding fragment thereof; and to provide a pharmaceutical use of the antibody or antigen-binding fragment thereof or the pharmaceutical composition.

On the basis of the specific epitope of the alpha chain of human IL-4R provided by the present disclosure, a further object of the present disclosure is to provide use of the epitope in preparing a binding agent of human IL-4R or a blocking agent of human IL-4/IL-13 signaling pathway, or use of the epitope in evaluating efficacy of the binding agent or the blocking agent.

The present disclosure provides the following technical solutions.

In one aspect, the present disclosure provides an antibody or antigen-binding fragment thereof that binds to human interleukin-4 receptor (IL-4R), the antibody or antigen-binding fragment thereof binding to an epitope which is located on the alpha chain of human IL-4R (IL-4Rα) and comprises one or more of amino acid residues D92, V94, D97, L67, L68, A96, H156, C207, Q63, and L64 in the amino acid sequence set forth in SEQ ID NO. 1.

Preferably, the epitope comprises one or more of amino acid residues D92, V94, and D97 in the amino acid sequence set forth in SEQ ID NO. 1.

According to particular embodiments of the present disclosure, the epitope comprises the following amino acid residues in the amino acid sequence set forth in SEQ ID NO. 1:
(1) D92;
(2) D97;
(3) D92, V94, and D97;
(4) D92 and V94;
(5) D92 and D97; or
(6) V94 and D97.

And/or, the antibody or antigen binding fragment thereof according to the present disclosure binds to an epitope of human IL-4Rα comprising the following amino acid residues in the amino acid sequence set forth in SEQ ID NO. 1:
(i) Q63 and L64.

Further, the antibody or antigen binding fragment thereof according to the present disclosure has no a cross-species binding activity to cynomolgus IL-4R. Preferably, the antibody or antigen binding fragment thereof does not bind to cynomolgus IL-4R; more preferably, does not bind to cynomolgus IL-4Rα; and further preferably, does not bind to the amino acid sequence set forth in SEQ ID NO. 2.

With respect to the cross-species binding activity above, the present disclosure provides an antibody or antigen-binding fragment thereof that binds to human interleukin-4 receptor (IL-4R), the antibody or antigen-binding fragment thereof binding to an epitope which is located on the alpha chain of human IL-4R (IL-4Rα) and comprises one or more of amino acid residues L67, L68, and A96 in the amino acid sequence set forth in SEQ ID NO. 1.

Preferably, the epitope comprises the following amino acid residues in the amino acid sequence set forth in SEQ ID NO. 1:
① L67 and L68;
② L67, L68, and A96; or
③ A96.

According to particular embodiments of the present disclosure, the epitope comprises the following amino acid residues in the amino acid sequence set forth in SEQ ID NO. 1:
(A) L67, L68, A96, H156, and C207;
(B) A96, H156, and C207;
(C) L67, L68, H156, and C207;
(D) L67, L68, A96, and C207; or
(E) L67, L68, A96, and H156.

Particularly preferably, the epitope according to the present disclosure comprises the following amino acid residues in the amino acid sequence set forth in SEQ ID NO. 1:
(P-1) D97, L67, and L68;
(P-2) D97, L67, L68, A96, and D92;
(P-3) D97, L67, L68, and D92;
(P-4) D97, Q63, and L64;
(P-5) D97, Q63, L64, and D92;
(P-6) D97, Q63, L64, D92, and A96; or
(P-7) D97, Q63, L64, L67, and L68.

Upon the detection of the binding of the antibodies provided in the present disclosure to the antigen IL-4Rα, it was found that the antibodies of the present disclosure had a binding activity to sIL-4Rα with an EC50 value no more than 50, 30, 20 or 10 ng/ml. It was also found that the antibodies of the present disclosure, at 100 µl × 2500 ng/ml, showed no binding activity to sIL-4Rα-97 coated at 100 µl × 0.5 µg/ml, the sIL-4Rα-97 being obtained upon the mutation at position 97 in sIL-4Rα from amino acid residue D to A. It was further found that the antibodies of the present disclosure, at 100 µl × 2500 ng/ml, showed no or a significant lower binding activity to the at least one mutated antigen selected from sIl-4Rα-QSMDH and sIl-4Rα-63/64 coated at 100 µl × 0.5 µg/ml. The binding activity was measured following the procedure described in the part "(I) Binding activity detection method" below, under subtitle **"DETAILED DESCRIPTION OF PREFERRED EMBODIMENTS"** of the present disclosure.

In addition, the antibodies provided in the present disclosure can block the binding of IL-4 or IL-13 to IL-4R, and thus can be used as blocking agents of the binding of IL-4 or IL-13 to IL-4R, or blocking agents of IL-4/IL-3 signaling pathway.

So with respect to the amino acid sequences of the domains comprised, the antibody or antigen-binding fragment thereof according to the present disclosure comprises a combination of heavy chain complementarity determining regions HCDR1, HCDR2, HCDR3 and light chain complementarity determining regions LCDR1, LCDR2, LCDR3 as follows:
(1) HCDR1, HCDR2, and HCDR3 set forth in SEQ ID NOs. 35, 36, and 37; and, LCDR1, LCDR2, and LCDR3 set forth in SEQ ID NOs. 53, 54, and 55;
(2) HCDR1, HCDR2, and HCDR3 set forth in SEQ ID NOs. 41, 42, and 43; and, LCDR1, LCDR2, and LCDR3 set forth in SEQ ID NOs. 59, 60, and 61;
(3) HCDR1, HCDR2, and HCDR3 set forth in SEQ ID NOs. 44, 45, and 46; and, LCDR1, LCDR2, and LCDR3 set forth in SEQ ID NOs. 56, 57, and 62;
(4) HCDR1, HCDR2, and HCDR3 set forth in SEQ ID NOs. 44, 47, and 46; and LCDR1, LCDR2, and LCDR3 set forth in SEQ ID NOs. 63, 64, and 65;
(5) HCDR1, HCDR2, and HCDR3 set forth in SEQ ID NOs. 48, 49, and 46; and, LCDR1, LCDR2, and LCDR3 set forth in SEQ ID NOs. 66, 67, and 68;
(6) HCDR1, HCDR2, and HCDR3 set forth in SEQ ID NOs. 44, 50, and 51; and, LCDR1, LCDR2, and LCDR3 set forth in SEQ ID NOs. 56, 57, and 69; or
(7) HCDR1, HCDR2, and HCDR3 set forth in SEQ ID NOs. 52, 36, and 37; and, LCDR1, LCDR2, and LCDR3 set forth in SEQ ID NOs. 53, 54, and 55.

The combinations of the light and heavy chain CDRs provided herein are derived from the particular antibodies or fragments thereof provided in the present disclosure. CDRs contained in the variable region amino acid sequences of a given antibody or antigen binding fragment thereof can be routinely determined by those skilled in the art. For example, according to particular embodiments of the present disclosure, an IMGT tool was used to define the CDRs in the amino acid sequences of the variable regions. Combinations of light and heavy chain CDRs defined by methods known in the art are all encompassed within the scope of the present disclosure.

In the antibody or antigen-binding fragment thereof according to the present disclosure, preferably, the heavy chain variable region comprises a sequence as follows: the amino acid sequence set forth in SEQ ID NO. 3, SEQ ID NO. 7, SEQ ID NO. 9, SEQ ID NO. 11, SEQ ID NO. 15, SEQ ID NO. 17, SEQ ID NO. 19, SEQ ID NO. 23, or SEQ ID NO. 27 or an amino acid sequence having at least 75% identity to the amino acid sequence; and/or, preferably, the light chain variable region comprises a sequence as follows: the amino acid sequence set forth in SEQ ID NO. 4, SEQ ID NO. 8, SEQ ID NO. 10, SEQ ID NO. 12, SEQ ID NO. 16, SEQ ID NO. 18, SEQ ID NO. 20, SEQ ID NO. 28, SEQ ID NO. 33, or SEQ ID NO. 34 or an amino acid sequence having at least 75% identity to the amino acid sequence.

According to particular embodiments of the present disclosure, in the antibody or antigen-binding fragment thereof according to the present disclosure:
(1) the heavy chain variable region comprises the amino acid sequence set forth in SEQ ID NO. 3 or an amino acid sequence having at least 75% identity to the amino acid sequence set forth in SEQ ID NO. 3; and, the light chain variable region comprises the amino acid sequence set forth in SEQ ID NO. 4 or an amino acid sequence having at least 75% identity to the amino acid sequence set forth in SEQ ID NO. 4;
(2) the heavy chain variable region comprises the amino acid sequence set forth in SEQ ID NO. 7 or an amino acid sequence having at least 75% identity to the amino acid sequence set forth in SEQ ID NO. 7; and, the light chain variable region comprises the amino acid sequence set forth in SEQ ID NO. 8 or an amino acid sequence having at least 75% identity to the amino acid sequence set forth in SEQ ID NO. 8;
(3) the heavy chain variable region comprises the amino acid sequence set forth in SEQ ID NO. 9 or an amino acid sequence having at least 75% identity to the amino acid sequence set forth in SEQ ID NO. 9; and, the light chain variable region comprises the amino acid sequence set forth in SEQ ID NO. 10 or an amino acid sequence having at least 75% identity to the amino acid sequence set forth in SEQ ID NO. 10;
(4) the heavy chain variable region comprises the amino acid sequence set forth in SEQ ID NO. 11 or an amino acid sequence having at least 75% identity to the amino acid sequence set forth in SEQ ID NO. 11; and, the light chain variable region comprises the amino acid sequence set forth in SEQ ID NO. 12 or an amino acid sequence having at least 75% identity to the amino acid sequence set forth in SEQ ID NO. 12;
(5) the heavy chain variable region comprises the amino acid sequence set forth in SEQ ID NO. 15 or an amino acid sequence having at least 75% identity to the amino acid sequence set forth in SEQ ID NO. 15; and, the light chain variable region comprises the amino acid sequence set forth in SEQ ID NO. 16 or an amino acid sequence having at least 75% identity to the amino acid sequence set forth in SEQ ID NO. 16;
(6) the heavy chain variable region comprises the amino acid sequence set forth in SEQ ID NO. 17 or an amino acid sequence having at least 75% identity to the amino acid sequence set forth in SEQ ID NO. 17; and, the light chain variable region comprises the amino acid sequence set forth in SEQ ID NO. 18 or an amino acid sequence having at least 75% identity to the amino acid sequence set forth in SEQ ID NO. 18;
(7) the heavy chain variable region comprises the amino acid sequence set forth in SEQ ID NO. 19 or an amino acid sequence having at least 75% identity to the amino acid sequence set forth in SEQ ID NO. 19; and, the light chain variable region comprises the amino acid sequence set forth in SEQ ID NO. 20 or an amino acid sequence having at least 75% identity to the amino acid sequence set forth in SEQ ID NO. 20;
(8) the heavy chain variable region comprises the amino acid sequence set forth in SEQ ID NO. 27 or an amino acid sequence having at least 75% identity to the amino acid sequence set forth in SEQ ID NO. 27; and, the light chain variable region comprises the amino acid sequence set forth in SEQ ID NO. 34 or an amino acid sequence having at least 75% identity to the amino acid sequence set forth in SEQ ID NO. 34;
(9) the heavy chain variable region comprises the amino acid sequence set forth in SEQ ID NO. 27 or an amino acid sequence having at least 75% identity to the amino acid sequence set forth in SEQ ID NO. 27; and, the light chain variable region comprises the amino acid sequence set forth in SEQ ID NO. 33 or an amino acid sequence having at least 75% identity to the amino acid sequence set forth in SEQ ID NO. 33;
(10) the heavy chain variable region comprises the amino acid sequence set forth in SEQ ID NO. 27 or an amino acid sequence having at least 75% identity to the amino acid sequence set forth in SEQ ID NO. 27; and, the light chain variable region comprises the amino acid sequence set forth in SEQ ID NO. 28 or an amino acid sequence having at least 75% identity to the amino acid sequence set forth in SEQ ID NO. 28;
(11) the heavy chain variable region comprises the amino acid sequence set forth in SEQ ID NO. 23 or an amino acid sequence having at least 75% identity to the amino acid sequence set forth in SEQ ID NO. 23; and, the light chain variable region comprises the amino acid sequence set forth in SEQ ID NO. 34 or an amino acid sequence having at least 75% identity to the amino acid sequence set forth in SEQ ID NO. 34;
(12) the heavy chain variable region comprises the amino acid sequence set forth in SEQ ID NO. 23 or an amino acid sequence having at least 75% identity to the amino acid sequence set forth in SEQ ID NO. 23; and, the light chain variable region comprises the amino acid sequence set forth in SEQ ID NO. 33 or an amino acid sequence having at least 75% identity to the amino acid sequence set forth in SEQ ID NO. 33; or
(13) the heavy chain variable region comprises the amino acid sequence set forth in SEQ ID NO. 23 or an amino acid sequence having at least 75% identity to the amino acid sequence set forth in SEQ ID NO. 23; and, the light chain variable region comprises the amino acid sequence set forth in SEQ ID NO. 28 or an amino acid sequence having at least 75% identity to the amino acid sequence set forth in SEQ ID NO. 28.

In particular, the antibody or antigen binding fragment thereof according to the present disclosure comprises at least a heavy chain variable region and a light chain variable region, both of which comprise the above CDRs and Framework Regions (FRs) in an arrangement as FR1-CDR1-FR2-CDR2-FR3-CDR3-FR4. Further alternatively, up to 25% difference in an amino acid sequence due to the "at least 75% identity" may be present in any framework region of the heavy chain variable region or the light chain variable region, or in any domain or sequence of the antibody or antigen binding fragment thereof according to the present disclosure other than the heavy chain variable region and the light chain variable region. The difference may be resulted from amino acid deletion, addition or substitution at any position, and the substitution may be conservative substitution or non-conservative substitution.

Preferably, the antibody or antigen-binding fragment thereof according to the present disclosure binds to human interleukin-4 receptor (IL-4R), preferably to the alpha chain of human IL-4R (IL-4Rα), more preferably to human soluble IL-4Rα (sIL-4Rα).

Preferably, the antibody according to the present disclosure can be a monoclonal antibody or a single chain antibody. Preferably, the antibody is a murine antibody, a chimeric antibody, or a fully or partially humanized antibody. Preferably, the antigen-binding fragment is a fragment of the antibody that is capable of specifically binding to the target. For example, the antigen binding fragment is an Fab fragment, an Fab' fragment, an F(ab')₂ fragment, or an Fv fragment (e.g., scFv) of the antibody.

Preferably, the antibody or antigen-binding fragment thereof further comprises a human or murine constant region, preferably a human or murine heavy chain constant region (CH) and/or a light chain constant region (CL). Further preferably, the antibody or antigen-binding fragment thereof comprises a heavy chain and a light chain. For example, the antibody is an immunoglobulin, in particular IgA, IgD, IgE, IgG, or IgM, for example a human IgA, IgD, IgE, IgG, or IgM, more preferably a human IgG1, IgG2, IgG3, or IgG4 subtype.

Preferably, the antibody or antigen-binding fragment thereof according to the present disclosure comprises a heavy chain constant region of an IgG, IgA, IgM, IgD, or IgE and/or a light chain constant region of a kappa or lambda type. For example, the heavy chain constant region is of an IgG (e.g., IgG1 or IgG4) type and the light chain constant region is of a kappa type. Further preferably, the heavy chain constant region of the monoclonal antibody comprises an amino acid sequence encoded by the nucleic acid sequence set forth in SEQ ID NO. 70 or SEQ ID NO. 71, or an amino acid sequence having at least 75% identity to the amino acid sequence encoded by the nucleic acid sequence set forth in SEQ ID NO. 70 or SEQ ID NO. 71; and the light chain constant region of the monoclonal antibody comprises an amino acid sequence encoded by the nucleic acid sequence set forth in SEQ ID NO. 72 or an amino acid sequence having at least 75% identity to the amino acid sequence encoded by the nucleic acid sequence set forth in SEQ ID NO. 72.

In the context of the present disclosure, the "at least 75% identity" is any percent identity between 75% and 100%, such as 75%, 80%, 85%, 90%, even 91%, 92%, 93%, 94%, 95%, 96%, 97%, 98%, or 99% identity.

In another aspect, the present disclosure also provides a nucleic acid molecule comprising a nucleotide sequence encoding a heavy chain variable region, a light chain variable region, a heavy chain, and/or a light chain of the antibody or antigen-binding fragment thereof according to the present disclosure.

The nucleic acid molecule according to the present disclosure may be cloned into a vector which in turn is transformed or transfected into a host cell. Thus, in a further aspect, the present disclosure also provides a vector comprising the nucleic acid molecule according to the present disclosure. The vector can be a eukaryotic expression vector, a prokaryotic expression vector, an artificial chromosome, a phage vector and the like.

The vector or nucleic acid molecule according to the present disclosure may be used to be transformed or transfected into a host cell for preservation or antibody expression or the like. Thus, in a further aspect, the present disclosure provides a host cell comprising the nucleic acid molecule and/or vector according to the present disclosure, or transformed or transfected with the nucleic acid molecule and/or vector according to the present disclosure. The host cell may be any prokaryotic or eukaryotic cell, such as a bacterial or insect, fungal, plant or animal cell.

The antibody or antigen-binding fragment thereof according to the present disclosure may be obtained using any method known in the art. For example, the heavy chain variable region and/or the light chain variable region of the antibody or the heavy chain and/or the light chain of the antibody may be obtained from the nucleic acid molecule according to the present disclosure, and then the antibody is obtained by assembling them with optional other domains of the antibody. Alternatively, the host cell according to the present disclosure is cultured under conditions that allow the host cell to express the heavy chain variable region and/or the light chain variable region of the antibody or the heavy chain and/or the light chain of the antibody and assemble them into an antibody. Optionally, the method may further include a step of recovering the produced antibody.

The antibody or antigen-binding fragment thereof, the nucleic acid molecule, the vector and/or the host cell according to the present disclosure may be comprised in a composition, more particularly in a pharmaceutical composition, such as a pharmaceutical preparation, for various purposes as actually needed. Thus, in a further aspect, the present disclosure also provides a composition, preferably a pharmaceutical composition, comprising the antibody or fragment thereof, the nucleic acid molecule, the vector and/or the host cell according to the present disclosure, and optionally a pharmaceutically acceptable excipient.

The pharmaceutical composition can be prepared into certain dosage forms, preferably for oral administration and parenteral administration (including subcutaneous, intramuscular and intravenous administration), including but not limited to a solid dosage form, a liquid dosage form, a semi-liquid dosage form, aerosol dosage form and suppository dosage form, etc. For example, dosage forms suitable for oral administration include tablet, capsule, granule, pulvis, pill, powder, lozenges, syrup and suspension; dosage forms suitable for parenteral administration include aqueous or non-aqueous solution and emulsion, for example, a subcutaneous injection.

Preferably, the pharmaceutical composition according to the present disclosure can be administered via a parenteral or non-parenteral (non-enteral) route which is therapeutically effective for an antibody drug. For example, the pharmaceutical composition according to the present disclosure is formed into a preparation containing a pharmaceutically acceptable excipient (e.g., vehicle) for systemic or topical administration.

In yet a further aspect, the present disclosure also provides use of the antibody or antigen-binding fragment thereof, the nucleic acid molecule, the vector, the host cell and/or the composition in the manufacture of a medicament. Preferably, the medicament is for the prevention, treatment or amelioration of a disease or disorder associated with human interleukin 4 receptor (IL-4R), or a disease or disorder associated with human interleukin 4 (IL-4) or human interleukin 13 (IL-13) signaling pathway.

In another aspect, the present disclosure also provides a method for preventing, treating, or ameliorating a disease or disorder, the method comprising administering (e.g., a therapeutically effective amount of) the antibody or antigen-binding fragment thereof, the nucleic acid molecule, the vector, the host cell, and/or the composition according to the present disclosure to a subject in need thereof. Preferably, the disease or disorder is associated with human interleukin 4 receptor (IL-4R), or with human interleukin 4 (IL-4) or human interleukin 13 (IL-13) signaling pathway, as described above.

In the above use or method provided by the present disclosure, the disease or disorder includes an autoimmune disease, an allergic disease, a tumor, or a cancer.

Preferably, the disease or disorder includes an autoimmune disease or an allergic disease, for example, dermatitis (e.g. atopic dermatitis), asthma (e.g. inflammatory asthma, such as inflammatory asthma type 2), chronic esophagitis (e.g. eosinophilic esophagitis), eczema, rhinitis (e.g. allergic rhinitis), nasal polyps (e.g. chronic rhinosinusitis with nasal polyps), conjunctivitis, inflammatory bowel disease (e.g. ulcerative colitis, and crohn's disease), inflammatory neuropathy, arthritis (e.g. rheumatoid arthritis), multiple sclerosis, lupus erythematosus, psoriasis, or insulin and non-insulin dependent diabetes mellitus, and other immune or allergy related diseases.

Alternatively, the disease or disorder includes a tumor or cancer, such as intestinal cancer, melanoma, skin cancer, breast cancer, uterine cancer, cervical cancer, endometrial cancer, ovarian cancer, testicular cancer, mesothelioma, prostate cancer, bladder cancer, anal cancer, glioblastoma, astrocytoma, liver cancer, kidney cancer, esophageal cancer, gastric cancer, lung cancer, head and neck cancer, myeloma, bone cancer, aids-related kaposi's sarcoma, hodgkin's or non-hodgkin's lymphoma, prostate cancer, prostate tumor, lymphoma, pancreatic cancer, and the like.

Preferably, the medicament is for the prevention, treatment or amelioration of one or more of the diseases or disorders as above.

Preferably, the subject is a mammal, preferably a primate; and more preferably, the subject is a human.

On the basis of the specific epitope of the alpha chain of human IL-4R (IL-4Rα) provided by the present disclosure, the present disclosure also provides use of the specific epitope in screening for a binding agent of human IL-4R or a blocking agent of human IL-4/IL-13 signaling pathway, or use of the specific epitope in evaluating efficacy of the binding agent or the blocking agent. As described above, the epitope comprises one or more of amino acid residues D92, V94, D97, L67, L68, A96, H156, C207, Q63, and L64 in the amino acid sequence set forth in SEQ ID NO. 1.

Preferably, the epitope comprises one or more of amino acid residues D92, V94, and D97 in the amino acid sequence set forth in SEQ ID NO. 1.

According to particular embodiments of the present disclosure, the epitope comprises the following amino acid residues in the amino acid sequence set forth in SEQ ID NO. 1:
(1) D92;
(2) D97;
(3) D92, V94, and D97;
(4) D92 and V94;
(5) D92 and D97; or
(6) V94 and D97.

And/or, the epitope comprises the following amino acid residues in the amino acid sequence set forth in SEQ ID NO. 1: (i) Q63 and L64.

And/or, the epitope comprises one or more of amino acid residues L67, L68, and A96 in the amino acid sequence set forth in SEQ ID NO. 1.

Preferably, the epitope comprises the following amino acid residues in the amino acid sequence set forth in SEQ ID NO. 1:
① L67 and L68;
(2) L67, L68, and A96; or
③ A96.

According to particular embodiments of the present disclosure, the epitope comprises the following amino acid residues in the amino acid sequence set forth in SEQ ID NO. 1:
(A) L67, L68, A96, H156, and C207;
(B) A96, H156, and C207;
(C) L67, L68, H156, and C207;
(D) L67, L68, A96, and C207; or
(E) L67, L68, A96, and H156.

Particularly preferably, the epitope according to the present disclosure comprises the following amino acid residues in the amino acid sequence set forth in SEQ ID NO. 1:
(P-1) D97, L67, and L68;
(P-2) D97, L67, L68, A96, and D92;
(P-3) D97, L67, L68, and D92;
(P-4) D97, Q63, and L64;
(P-5) D97, Q63, L64, and D92;
(P-6) D97, Q63, L64, D92, and A96; or
(P-7) D97, Q63, L64, L67, and L68.

Based on the uses of the specific epitope as above, the present disclosure provides a method for screening for a binding agent that specifically binds to human interleukin 4 receptor (IL-4R) or a blocking agent that specifically blocks human interleukin 4 (IL-4) or human interleukin 13 (IL-13) signaling pathway, the method comprising the steps as follows: contacting an agent to be screened with human IL-4R or part thereof, and then detecting whether the agent is capable of binding to the epitope according to the present disclosure. When the agent is detected capable of binding to the epitope according to the present disclosure, it is identified as the binding agent or blocking agent.

Alternatively, the present disclosure also provides a method for evaluating efficacy of an agent, the method comprising the steps as follows: contacting the agent to be evaluated with human interleukin 4 receptor (IL-4R) or part thereof, and then detecting whether the agent is capable of binding to the epitope according to the present disclosure. When the agent is detected capable of binding to the epitope according to the present disclosure, it is identified as a binding agent that specifically binds to human interleukin 4 receptor (IL-4R) or a blocking agent that specifically blocks human interleukin 4 (IL-4) or human interleukin 13 (IL-13) signaling pathway, and accordingly has an efficacy in preventing, treating, or ameliorating a disease or disorder described in the present disclosure.

In the method for screening for a binding agent or a blocking agent or in the method for evaluating efficacy of an agent according to the present disclosure, preferably, the agent to be screened or evaluated is an antibody, such as a polyclonal antibody or a monoclonal antibody. More preferably, the antibody is obtained through immunizing an animal with human IL-4R or part thereof as an immunogen.

In the method for screening for a binding agent or a blocking agent or in the method for evaluating efficacy of an agent, preferably, the detecting whether the agent is capable of binding to the epitope according to the present disclosure may be performed by: mutating one or more amino acid residues comprised by the epitope according to the present disclosure through site-directed mutagenesis of human IL-4R or part thereof, comparing the binding activity or affinity of the agent to the human IL-4R or part thereof after the mutation with that before the mutation, and identifying the agent capable of binding to the epitope according to the present disclosure, if the binding activity or affinity of the agent to the mutated human IL-4R or part thereof is significantly lower or even lost.

Specifically, the method for screening for a binding agent or a blocking agent or the method for evaluating efficacy of an agent according to the present disclosure may include the steps as follows:
1) mutating one or more amino acid residues comprised by the epitope according to the present disclosure through site-directed mutagenesis of human interleukin 4 receptor (IL-4R) or part thereof, to obtain a mutant of the human IL-4R or part thereof;
2) contacting an agent to screened or an agent to be evaluated with the human IL-4R or part thereof and the mutant of the human IL-4R or part thereof respectively, and detecting the binding activity or affinity thereto respectively;
3) identifying the agent capable of binding to the epitope according to the present disclosure, if the binding activity or affinity of the agent to the mutated human IL-4R or part thereof is significantly lower or even lost compared with the binding activity or affinity of the agent to the human IL-4R or part thereof.

Those skilled in the art can routinely determine whether the binding activity or affinity is significantly lower or lost. For example, if the binding activity or affinity of the agent to a mutant of human IL-4R or part thereof is a tenth of the binding activity or affinity before the mutation, it can be determined that the binding activity or affinity is significantly lower.

Preferably, in step 1), one or more amino acid residues comprised by the epitope are mutated to Alanine or to an amino acid residue of the cynomolgus interleukin 4 receptor at the corresponding position, to obtain a mutant of human IL-4R or part thereof.

Preferably, the part of human IL-4R used in the method according to the present disclosure is the alpha chain of human IL-4R, further human sIL-4R alpha. Preferably, therefore, the method is performed using human IL-4Rα and a mutant of human IL-4Rα is obtained. Further, the method can be performed using human sIL-4Rα and one or more of the mutants of human sIL-4Rα listed in Table 3 below are used as the mutant in step 1).

In the present disclosure, human IL-4Rα refers to the sequence under NCBI accession number NP_000409.1; the amino acid sequence of human sIL-4Rα is set forth in SEQ ID NO. 1; and the amino acid sequence of cynomolgus sIL-4Rα is set forth in SEQ ID NO. 2.

Experiments prove that the antibody or antigen binding fragment thereof according to the present disclosure binds to an epitope located on the alpha chain (IL-4Rα) of human interleukin 4 receptor (IL-4R), and the epitope comprises one or more of the amino acid residues D92, V94, D97, L67, L68, A96, H156, C207, Q63, and L64 in the amino acid sequence set forth in SEQ ID NO. 1. The epitope of sIL-4Rα in immune responses was less studied previously, and in the present disclosure critical positions in the binding of anti-IL-4R antibodies to IL-4R are defined for the first time.

Accordingly, the present disclosure provides antibodies against human IL-4R, particularly IL-4Rα, binding to a novel epitope of IL-4R. The antibodies can be novel, useful, and effective antibodies against human IL-4R, particularly IL-4Rα. Furthermore, the present disclosure provides an alternative approach to the development of potential antibodies or drugs against IL-4R, particularly human IL-4Rα, by elucidating the critical positions in the binding of the antibodies provided by the present disclosure to human IL-4Rα.

### BRIEF DESCRIPTION OF THE DRAWINGS

Embodiments of the present disclosure are described in detail below with reference to the attached figures, in which:
FIG. 1 shows the alignment of human IL-4Rα (Sequence_1; SEQ ID NO. 1) and cynomolgus IL-4Rα (Sequence_2; SEQ ID NO. 2).
FIG. 2 shows the stability of the antibodies of the present disclosure, in which panel 2A shows the result after storage at 40 °C for 2 weeks; and panel 2B shows the result after storage at 40 °C for 4 weeks.

### DETAILED DESCRIPTION OF PREFERRED EMBODIMENTS

In the present disclosure, the term "epitope" refers to the amino acid residue(s) of an antigen corresponding to the binding site when an antibody or a fragment thereof binds to the antigen and the corresponding position(s) of the amino acid residue(s) in the sequence of the antigen; also, the mutation of the amino acid residue(s) of the epitope will result in a decrease of more than 10² ng/ml of binding activity or a loss of binding activity as measured following the procedure described in "(I) Binding activity detection method" below.

The following experimental procedures or definitions are involved in the present disclosure. It should be noted that the present disclosure can also be implemented by other conventional techniques in the art, and is not limited to the following experimental procedures.

### (I) Binding activity detection method

### 1. Preparation of reagents

Preparation of antigen: the antigen was prepared into a 100 µg/ml solution in PBS, which was stored in a refrigerator at -20 °C after being subpackaged.

Preparation of antibody to be detected: the antibody to be detected was diluted with PBS containing 1% BSA, to concentrations of 0, 0.032, 0.16, 0.8, 4, 20, 100, 500, and 2500 ng/ml desired.

Preparation of secondary antibody working solution: the secondary antibody stock solution (Goat Anti-Human IgG-Fc Secondary Antibody (HRP), sino biological, Cat Number: SSA001) was diluted 16000-fold with PBS containing 1% BSA to obtain a working solution. For example, 1 µl of the secondary antibody stock solution was added to 15999 µl of PBS containing 1% BSA, and the mixture obtained was mixed well by repeatedly inverting up and down. The secondary antibody working solution of a volume as desired can be prepared by scaling up according to the experiment requirement.

### 2. ELISA detection

50 µl of the 100 µg/ml antigen solution was added into 9.95 ml of PBS, and the mixture obtained was mixed well by inverting up and down to obtain 0.5 µg/ml antigen coating solution. The prepared antigen-coating solution was pipetted using a 12-channel pipette (RAININ) and added to a 96-well plate (Corning), 100 µl per well. The 96-well plate was wrapped with preservative film (or covered) and incubated overnight in a refrigerator at 4 °C. The next day, the 96-well plate was removed, the solution therein was discarded, and the plate was gently patted dry on a clean paper towel. PBS was added to the 96-well plate, 300 µl per well. After the plate was allowed to stand at room temperature for 3 min, the solution therein was discarded, and the plate was gently patted dry on a clean paper towel. Then the plate was washed 3 times. PBS containing 2% BSA was added to the 96-well plate, 300 µl per well. The 96-well plate was wrapped with preservative film (or covered) and incubated for 2 h in a constant-temperature incubator at 37 °C. The 96-well plate was removed, the solution therein was discarded, and the 96-well plate was gently patted dry on a clean paper towel. PBS containing 0.05% Tween-20 (Beyotime, Cat Number: ST825) was added to the 96-well plate, 300 µl per well. After the plate was allowed to stand at room temperature for 3 min, the solution therein was discarded, and the plate was gently patted dry on a clean paper towel. Then the plate was washed 3 times.

The antibody to be detected which was diluted sequentially as above was added into corresponding wells respectively, 100 µl per well and each sample was replicated in three wells. The 96-well plate was wrapped with preservative film (or covered) and incubated for 1 h in a constant-temperature incubator at 37 °C. The 96-well plate was removed, the solution therein was discarded, and the 96-well plate was gently patted dry on a clean paper towel. PBS containing 0.05% Tween-20 was added to the 96-well plate, 300 µl per well. After the plate was allowed to stand at room temperature for 3 min, the solution therein was discarded, and the plate was gently patted dry on a clean paper towel. Then the plate was washed 3 times. The secondary antibody working solution was added to the 96-well plate, 100 µl per well. The 96-well plate was wrapped with preservative film (or covered) and incubated for 1 h in a constant-temperature incubator at 37 °C. Then the 96-well plate was removed, the solution therein was discarded, and the plate was gently patted dry on a clean paper towel. PBS containing 0.05% Tween-20 was added to the 96-well plate, 300 µl per well. After the plate was allowed to stand at room temperature for 3 min, the solution therein was discarded, and the plate was gently patted dry on a clean paper towel. Then the plate was washed 3 times. TMB substrate solution (SurModics, Cat Number: TMDS-1000-01) was added to the 96-well plate, 100 µl per well. Then the plate was incubated for 5 min in a constant-temperature incubator at 37 °C and then 2 M H₂SO₄ solution was immediately added to the 96-well plate to stop the reaction. The 96-well plate was placed in flexstation 3 (Molecular Devices), and the OD450 value was read and data was collected for calculation and analysis.

### (II) Cell functional activity detection method

### 1. Preparation of reagents

Preparation of human IL-4 (Invivogen, Cat Number: rhIL-4): human IL-4 was prepared into a 100 µg/ml solution in PBS, which was stored in a refrigerator at -20 °C after being subpackaged.

Preparation of human IL-13 (Invivogen, Cat Number: rhIL-13): human IL-13 was prepared into a 100 µg/ml solution in PBS, which was stored in a refrigerator at -20 °C after being subpackaged.

Preparation of Quanti-blue (I) solution: a pack of Quanti-blue^{™} powder was poured into a sterile 250 mL bottle, into which 100 mL of sterile water were added then. The mixture obtained was mixed gently, and the bottle was placed in a bathing at 37 °C for 30 min. When the powder was completely dissolved, the bottle was placed in a refrigerator at 4 °C in dark overnight. Afterwards, the solution in it was subpackaed, 10 mL per tube, and stored in dark at -20 °C. The solution can be stored for 6 months.

Preparation of antibody to be detected: the antibody to be detected was diluted in DMEM medium (HyClone, Cat number SH30022.01) containing 10% FBS (Hyclone, Cat number SV30184.02), to concentrations desired (1000, 200, 40, 8, 1.6, 0.32, 0.064, 0.0128, 0.00256, and 0 ng/ml).

### 2. Cell culture

Frozen HEK-Blue^{™} IL-4/IL-13 cells (Invivogen, Cat number hkb-il413) were removed from liquid nitrogen and rapidly lysed by gentle shaking in 37 °C water bath. The dissolved cell suspension was transferred into a 15 ml centrifuge tube, DMEM medium (containing 10% FBS, 10 µg/ml Blasticidin, 100 µg/ml Zeocin, and 100 µg/ml Normocin) was added into the tube to 10 ml, and then the tube was centrifuged for 5 min at 800 rpm. The supernatant was sucked out, and the remained cell pellets were washed once. 10 ml of DMEM medium were added into the cells to adjust the cell density to 1×10⁵ - 1 ×10⁶ cells/ml. Then the cell suspension was transferred into a T75 cell culture bottle (Nunc), which was then placed in a 37 °C, 5% CO₂ incubator (Thermo) for stationary culture. Every 2-3 days the cell suspension was taken and centrifuged at 800 rpm for 5 min, and the cells obtained were re-suspended in 10 ml of the culture medium, from which 1×10⁶ cells were counted and transferred into a new T75 cell culture bottle, into which simultaneously the culture medium was supplemented to 10 ml. The cells were passaged for 2-3 passages continuously to achieve a good growth state of the cells (the cells were transparent, spindle, and adherent) before the experiment below could be performed.

### 3. Blocking experiment

The T75 cell bottle containing the cells with a good growth state was taken, the supernatant therein was discarded, and the adherent cells were gently detached with 10 ml of PBS. The detached cells were transferred into a 15 ml centrifuge tube, which was then centrifuged for 5 min at 800 rpm, and the cell pellets were re-suspended in 10 ml of PBS. The cells were centrifuged for 5 min at 800 rpm again, the supernatant was discard, and the remained cell pellets were re-suspended in 5 ml of DMEM medium containing 10% FBS, and after cell counting, the medium was supplemented to adjust the cell density to 6.6×10⁵ cells/ml. The cell suspension was added to a 384 well plate, 30 µl per well. The antibody to be detected at different concentrations prepared as above was added to the cells in the wells of the 384 well plate, 10 µl per well (each sample was replicated in three wells). Human IL-4 or human IL-13 was diluted to 2.5 ng/ml in DMEM medium containing 10% FBS, and 2.5 ng/ml human IL-4 or human IL-13 was added to corresponding wells of the 384 well plate, 10 µl per well, so that the final number of cells in each well was 2×10⁴, the final concentration of the human IL-4 or human IL-13 was 0.5 ng/ml, and the final volume in each well of the 384 well plate was 50 µl. The 384-well plate was placed in a 37 °C, 5% CO₂ incubator for stationary culture of the cells.

### 4. Data statistics

After the 384 well plate was incubated for 22 h in the 5% CO₂ incubator, 45 µl of the Quanti-blue solution were added to each well of a new 384 well plate. The supernatant in each well of the plate which had been incubated for 22 h were pipetted and added into a corresponding well of the new 384 well plate, 5 µl per well, which was then incubated at 37 °C for 60 min. The 384 well plate was placed in flexstation 3, and the OD650 value was read and data was collected for calculation and analysis.

The present disclosure is illustrated below with reference to specific examples. It will be understood by those skilled in the art that these examples are merely illustrative of the present disclosure and do not limit the scope of the present disclosure in any way.

The experimental procedures in the following examples are all conventional, unless otherwise specified. The raw materials and reagents used in the following examples are all commercially available products, unless otherwise specified, in which:
Human sIL-4Rα: NP_000409.1, Met1-His232, set forth in SEQ ID NO. 1;
Cynomolgus sIL-4Rα: EHH60265.1, Met1-Arg232, set forth in SEQ ID NO. 2.

### EXAMPLE 1 Preparation of murine, chimeric, and humanized antibodies

Ten mice were immunized with human sIL-4Rα (SEQ ID NO. 1); blood of the mice was collected, and detected following the procedures described in "(I) Binding activity detection method" and "(II) Cell functional activity detection method". According to a comprehensive analysis on the results of the two detections, two mice exhibited best results were selected for fusion respectively.

Supernatants obtained from different fusions were detected following the procedures described in "(I) Binding activity detection method" and "(II) Cell functional activity detection method" again. According to a comprehensive analysis on the results of the two detections, parent clones exhibited highest acitivity were selected for subcloning. Through detections following the procedures described in "(I) Binding activity detection method" and "(II) Cell functional activity detection method", 14 murine monoclonal antibodies were finally screened out. The 14 monoclonal antibodies were sequenced, and antibodies having 9 pairs of new sequences were obtained. Amino acid sequences of the heavy chain and light chain variable regions of the antibodies are shown below; and CDRs therein defined by an IMGT tool are underlined.
Y0188-1
   Heavy chain variable region (SEQ ID NO: 3; CDRs sequentially: SEQ ID NO: 35, SEQ ID NO: 36, SEQ ID NO: 37) 
EVQLVESGGGLVQPKGSLKLSCAASGFTFNTYGMHWVRQAPGKGLEWVAHIRSKSS
NYATYYADSVKDRFTISRDDSQSMLYLQMNNLKTEDTAMYYCVRWFRAMDYWGQ
GTSVTVSS
   Light chain variable region (SEQ ID NO: 4; CDRs sequentially: SEQ ID NO: 53, SEQ ID NO: 54, SEQ ID NO: 55) 
DIVMTQSHKFMSTSVGDRVSITCKASQDVSTAVAWYQEKPGQSPKLLIYWASTRHTG
VPDRFTGSGSGTDYTLTISSVQAEDLALYYCQQHYSTPLTFGAGTKLELK
Y0188-2
   Heavy chain variable region (SEQ ID NO: 5; CDRs sequentially: SEQ ID NO: 38, SEQ ID NO: 39, SEQ ID NO: 40) 
EVQLIESGGGLVQPKGSLKLSCAASGFTFNMYAMDWVRQAPGKGLEWVARIRSKGS
NFETNYADSVKDRFTISRDDSQSMVYLQMINLKTEDTAMYYCVRHRGGAWFAYWG
QGTLVSVSA
   Light chain variable region (SEQ ID NO: 6; CDRs sequentially: SEQ ID NO: 56, SEQ ID NO: 57, SEQ ID NO: 58) 
DIVVTQSPASLAVSLGQRATISCRASKSVSTSGYSYMHWYQQKPGQPPKLLIYLASNLE
SGVPARFSGSGSGTDFTLNIHPVEEEDVAIYYCQHSRELPLTFGAGTKLELK
Y0188-3
   Heavy chain variable region (SEQ ID NO: 7; CDRs sequentially: SEQ ID NO: 41, SEQ ID NO: 42, SEQ ID NO: 43) 
QVQLVETGGGLVRPGNSLKLSCVTSGFTFSNYRMHWLRQPPGKRLEWIAVITVKSNN
YGANYAESVKGRFAISRDDSKSSVYLEMNRLREEDTATYFCSRERAYGNPFDYWGQG
TTLTVSS
   Light chain variable region (SEQ ID NO: 8; CDRs sequentially: SEQ ID NO: 59, SEQ ID NO: 60, SEQ ID NO: 61) 
DIQMTQSPSSLSASLGERVSLTCRASQEISGYLSWLQQKPDGTIKRLIYAASTLDSGVPK
RFSGSRSGSDYSLTISSLESEDFADYYCLQYGSYPYTFGGGTKLEIK
Y0188-4
   Heavy chain variable region (SEQ ID NO: 9; CDRs sequentially: SEQ ID NO: 44, SEQ ID NO: 45, SEQ ID NO: 46) 
EVQLVESGGGLVQPKGSLKLSCAASGFTFNMYAMNWVRQAPGQGLEWVARIRSKSN
NYATYYADSVKDRFIISRDDSESMVYLQMSNLRAADTAMYYCVRHLRAMDYWGQG
TSVTVSS
   Light chain variable region (SEQ ID NO: 10; CDRs sequentially: SEQ ID NO: 56, SEQ ID NO: 57, SEQ ID NO: 62) 
DIVLTQSPASLTVSLGQRATISCRASKSVSTSGYSYMHWYQQKPGQPPKLLIYLASNLE
SGVPARFSGSGSGTDFTLNIHPVEEEDAATYYCQHSRELPITFGSGTKLEIK
Y0188-6
   Heavy chain variable region (SEQ ID NO: 11; CDRs sequentially: SEQ ID NO: 44, SEQ ID NO: 47, SEQ ID NO: 46) 
EVQLVESGGGLVQPKGSLKLSCAASGFSFNMYAMNWVRQAPGKGLEWVARIRTKSN
HYSTYYADSVKDRFTISRDDSASMFYLQMNNLKTEDTAMYFCVRHLRAMDYWGQG
TSVTVSS
   Light chain variable region (SEQ ID NO: 12; CDRs sequentially: SEQ ID NO: 63, SEQ ID NO: 64, SEQ ID NO: 65) 
DIVLTQSPASLVVSLGQRATISCRASQSVSTSGYSYMHWYQQKPGQPPKLLIYLASNV
QSGVPARFSGSGSGTDFTLNIHPVEEEDVATYYCHHNRDLPFTFGSGTKLEIK
Y0188-8
   Heavy chain variable region (SEQ ID NO: 13; CDRs sequentially: SEQ ID NO: 38, SEQ ID NO: 39, SEQ ID NO: 40) 
EVQLIESGGGLVQPKGSLKLSCAASGFTFNMYAMDWVRQAPGKGLEWVARIRSKGS
NFETNYADSVKDRFTISRDDSQSMVYLQMNNLKTEDTAMYYCVRHRGGAWFAYWG
QGTLVTVSA
   Light chain variable region (SEQ ID NO: 14; CDRs sequentially: SEQ ID NO: 56, SEQ ID NO: 57, SEQ ID NO: 58) 
DIVVTQSPASLAVSLGQRATISCRASKSVSTSGYSYMHWYQQKPGQPPKLLIYLASNLE
SGVPARFSGSGSGTDFTLNIHPVEEEDVAIYYCQHSRELPLTFGAGTKLELK
Y0188-9
   Heavy chain variable region (SEQ ID NO: 15; CDRs sequentially: SEQ ID NO: 48, SEQ ID NO: 49, SEQ ID NO: 46) 
EVQLVESGGGLVRPKGSLKLSCAASGFSFNTYAMNWVRQAPGKGLEWIVWIRSKSHN
YATYYADSVKDRFTISRDDSESMLYLQMNNLKTEDTAMYYCVRHLRAMDYWGQGT
SVTVSS
   Light chain variable region (SEQ ID NO: 16; CDRs sequentially: SEQ ID NO: 66, SEQ ID NO: 67, SEQ ID NO: 68) 
DIVLTQSPASLAVSLGQRATISCRASKSVSASGYSYMHWYQQKPGQPPKLLIYLASNL
QSGVPARFSGSGSGTDFTLNIHPVEEEDAATYYCQHSRELPPTFGGGTKLEIK
Y0188-10
   Heavy chain variable region (SEQ ID NO: 17; CDRs sequentially: SEQ ID NO: 44, SEQ ID NO: 50, SEQ ID NO: 51) 
EVRLVESGGGLVQPKGSLKLSCEASGFSFNMYAMNWVRQAPGKGLEWITHIRSKSNN
YATYYADSVKDRFIISRDDSESMVYLQMNNLKTEDTAMYYCVRLLRALDYWGQGTS
VTVSS
   Light chain variable region (SEQ ID NO: 18; CDRs sequentially: SEQ ID NO: 56, SEQ ID NO: 57, SEQ ID NO: 69) 
DIVLTQSPASLAVFLGQRATISCRASKSVSTSGYSYMHWYQQKAGQPPKLLIYLASNL
ESGVPARFSGSGSGTDFTLNIHPVEEEDAATYYCHHSRELPITFGSGTKLEMK
Y0188-14
   Heavy chain variable region (SEQ ID NO: 19; CDRs sequentially: SEQ ID NO: 52, SEQ ID NO: 36, SEQ ID NO: 37) 
EVQLVESGGGLVQPKGSLKLSCAASGFTFNMYGMHWVRQAPGKGLEWVAHIRSKSS
NYATYYADSVKDRLTISRDDSQSMLYLQMNNLKTEDTAMYYCVRWFRAMDYWGQ
GTSVTVSS
   Light chain variable region (SEQ ID NO: 20; CDRs sequentially: SEQ ID NO: 53, SEQ ID NO: 54, SEQ ID NO: 55) 
DIVMTQSHKFMSTSVGDRVSITCKASQDVSTAVAWYQEKPGQSPKLLIYWASTRHTG
VPDRFTGSGSGTDYTLTISSVQAEDLALYYCQQHYSTPLTFGAGTKLELK
9 chimeric antibodies were constructed using the variable region sequences above, and using the sequence set forth in SEQ ID NO. 71 as the sequence encoding the heavy chain constant region and the sequence set forth in SEQ ID NO. 72 as the sequence encoding the light chain constant region. The 9 chimeric antibodies were expressed and purified, and named as the names of the murine antibodies plus Q. IC50 values of the chimeric antibodies were detected following the procedure described in "(II) Cell functional activity detection method", and the affinity (KD) of the chimeric antibodies for human sIL-4Rα was measured by a BIACORE instrument. The results are shown in Table 1 below.

**Table 1. Detection results of cell functional activity and affinity of the chimeric antibodies**

| Chimeric antibody | IC50 (ng/ml) (blocking IL-4) | IC50 (ng/ml) (blocking IL-13) | KD (M) |
|---|---|---|---|
| Y0188-1Q | 242.6 | 1146 | 2.96E-09 |
| Y0188-2Q | 283.2 | 1279 | 2.31E-09 |
| Y0188-3Q | 231.2 | 745.3 | 3.20E-09 |
| Y0188-4Q | 1309.0 | 3088 | 4.91E-09 |
| Y0188-6Q | 121.6 | 999.3 | 4.05E-09 |
| Y0188-8Q | 239.7 | 583.8 | 2.72E-09 |
| Y0188-9Q | 357.8 | 1546 | 3.67E-09 |
| Y0188-10Q | 130.9 | 808.3 | 4.17E-09 |
| Y0188-14Q | 30.18 | 55.16 | 2.91E-09 |

The chimeric antibody (Y0188-14Q) from clone No. 14 was selected and humanized to obtain 7 humanized sequences of the heavy chain variable region and 7 humanized sequences of the light chain variable region. Humanized amino acid sequences of the heavy chain and light chain variable regions are shown below; and CDRs therein defined by an IMGT tool are underlined.
Heavy chain variable region:
   > HV3-15-14H (SEQ ID NO: 21; CDRs sequentially: SEQ ID NO: 52, SEQ ID NO: 36, SEQ ID NO: 37) 
EVQLVESGGGLVKPGGSLRLSCAASGFTFSMYGMHWVRQAPGKGLEWVGHIRSKSSN
YATYYADSVKDRFTISRDDSKNTLYLQMNSLKTEDTAVYYCTTWFRAMDYWGQGTLV
TVSS
   >HV3-48-14H (SEQ ID NO: 22; CDRs sequentially: SEQ ID NO: 52, SEQ ID NO: 36, SEQ ID NO: 37) 
EVQLVESGGGLVQPGGSLRLSCAASGFTFSMYGMHWVRQAPGKGLEWVSHIRSKSSN
YATYYADSVKDRFTISRDNAKNSLYLQMNSLRAEDTAVYYCARWFRAMDYWGQGTL
VTVSS
   >HV3-73*2-14H (SEQ ID NO: 23; CDRs sequentially: SEQ ID NO: 52, SEQ ID NO: 36, SEQ ID NO: 37) 
EVQLVESGGGLVQPGGSLKLSCAASGFTFSMYGMHWVRQASGKGLEWVGHIRSKSSN
YATYYADSVKDRFTISRDDSKNTAYLQMNSLKTEDTAVYYCTRWFRAMDYWGQGTLV
TVSS
   >HV3-72-14H (SEQ ID NO: 24; CDRs sequentially: SEQ ID NO: 52, SEQ ID NO: 36, SEQ ID NO: 37) 
EVQLVESGGGLVQPGGSLRLSCAASGFTFSMYGMHWVRQAPGKGLEWVGHIRSKSSN
YATYYADSVKDRFTISRDDSKNSLYLQMNSLKTEDTAVYYCARWFRAMDYWGQGTL
VTVSS
   >Y01-14H (SEQ ID NO: 25; CDRs sequentially: SEQ ID NO: 52, SEQ ID NO: 36, SEQ ID NO: 37) 
EVQLVESGGGLVQPGGSLRLSCAASGFTFSMYGMHWVRQAPGKGLEWVSHIRSKSSN
YATYYADSVKDRFTISRDNAKNSLYLQMNSLRAEDTAVYYCARWFRAMDYWGQGTL
VTVSS
   >162-14H (SEQ ID NO: 26; CDRs sequentially: SEQ ID NO: 52, SEQ ID NO: 36, SEQ ID NO: 37) 
EVQLVESGGGLEQPGGSLRLSCAGSGFTFRMYGMHWVRQAPGKGLEWVSHIRSKSSN
YATYYADSVKDRFTISRDNSKNTLYLQMNSLRAEDTAVYYCAKWFRAMDYWGQGTT
VTVSS
   >VH73-14H (SEQ ID NO: 27; CDRs sequentially: SEQ ID NO: 52, SEQ ID NO: 36, SEQ ID NO: 37) EVQLVESGGGLVQPGGSLKLSCAASGFTFSMYGMHWVRQASGKGLEWVGHIRSKSSN 
YATYYADSVKDRFTISRDDSKNTAYLQMNSLKTEDTAVYYCTRWFRAMDYWGQGTT
VTVSS
Light chain variable region:
   >Y01-14L (SEQ ID NO: 28; CDRs sequentially: SEQ ID NO: 53, SEQ ID NO: 54, SEQ ID NO: 55) 
EIVLTQSPGTLSLSPGERATLSCKASQDVSTAVAWYQQKPGQAPRLLIYWASTRHTGIPD
RFSGSGSGTDFTLTISRLEPEDFAVYYCQQHYSTPLTFGQGTKVEIK
   >164-14L (SEQ ID NO: 29; CDRs sequentially: SEQ ID NO: 53, SEQ ID NO: 54, SEQ ID NO: 55) 
DIVMTQSPLSLPVTPGEPASISCKASQDVSTAVAWYLQKSGQSPQLLIYWASTRHTGVP
DRFSGSGSGTDFTLKISRVEAEDVGFYYCQQHYSTPLTFGQGTKLEIK
   >KV4-14L (SEQ ID NO: 30; CDRs sequentially: SEQ ID NO: 53, SEQ ID NO: 54, SEQ ID NO: 55) 
DIVMTQSPDSLAVSLGERATINCKASQDVSTAVAWYQQKPGQPPKLLIYWASTRHTGVP
DRFSGSGSGTDFTLTISSLQAEDVAVYYCQQHYSTPLTFGGGTKVEIK
   > KV1-27-14L (SEQ ID NO: 31; CDRs sequentially: SEQ ID NO: 53, SEQ ID NO: 54, SEQ ID NO: 55) 
DIQMTQSPSSLSASVGDRVTITCKASQDVSTAVAWYQQKPGKVPKLLIYWASTRHTGV
PSRFSGSGSGTDFTLTISSLQPEDVATYYCQQHYSTPLTFGGGTKVEIK
   >KV1-9-14L (SEQ ID NO: 32; CDRs sequentially: SEQ ID NO: 53, SEQ ID NO: 54, SEQ ID NO: 55) 
DIQLTQSPSFLSASVGDRVTITCKASQDVSTAVAWYQQKPGKAPKLLIYWASTRHTGVP
SRFSGSGSGTEFTLTISSLQPEDFATYYCQQHYSTPLTFGGGTKVEIK
   > KV1-NL1-14L (SEQ ID NO: 33; CDRs sequentially: SEQ ID NO: 53, SEQ ID NO: 54, SEQ ID NO: 55) 
DIQMTQSPSSLSASVGDRVTITCKASQDVSTAVAWYQQKPGKAPKLLLYWASTRHTGV
PSRFSGSGSGTDYTLTISSLQPEDFATYYCQQHYSTPLTFGGGTKVEIK
   > KV1D-43-14L (SEQ ID NO: 34; CDRs sequentially: SEQ ID NO: 53, SEQ ID NO: 54, SEQ ID NO: 55) 
AIRMTQSPFSLSASVGDRVTITCKASQDVSTAVAWYQQKPAKAPKLFIYWASTRHTGVP
SRFSGSGSGTDYTLTISSLQPEDFATYYCQQHYSTPLTFGGGTKVEIK
49 humanzied antibodies were constructed using the humanized sequences of the light and heavy chain variable regions paired, and using the sequence set forth in SEQ ID NO. 71 as the sequence encoding the heavy chain constant region and the sequence set forth in SEQ ID NO. 72 as the sequence encoding the light chain constant region. The humanized antibodies were expressed, IC50 values of the antibodies were detected following the procedure described in "(II) Cell functional activity detection method", and the cell functional activity (KD) of the antibodies was confrimed by a BIACORE instrument. Preferred 6 combinations of the variable regions were obtained. The results are shown in Table 2 below.

**Table 2. Detection results of cell functional activity of the humanized antibodies**

| Humanzied antibody | IC50 (ng/ml) (blocking IL-4) | KD (M) |
|---|---|---|
| B4 (VH73-14H/KV1D-43-14L) | 61.95 | 6.42E-09 |
| B5 (VH73-14H/KV1-NL1-14L) | 25.06 | 2.31E-09 |
| B7 (VH73-14H/Y01-14L) | 122.0 | 5.45E-09 |
| E4 (HV3-73*2-14H/KV1D-43-14L) | 41.37 | 6.12E-09 |
| E5 (HV3-73*2-14H/KV1-NL1-14L) | 11.80 | 1.85E-09 |
| E7 (HV3-73*2-14H/Y01-14L) | 45.75 | 4.72E-09 |

### EXAMPLE 2 Study on binding epitope

2.1 Species differences in the binding of antibodies to human IL-4Rα and cynomolgus IL-4Rα Cynomolgus sIL-4Rα (EHH60265.1, Met1-Arg232; SEQ ID NO. 2) was used as the antigen, and the binding activity of the antibodies to the antigen was detected following the procedure described in "(I) Binding activity detection method".

### 2.2 Selection of the positions in antigen sequence to be mutated

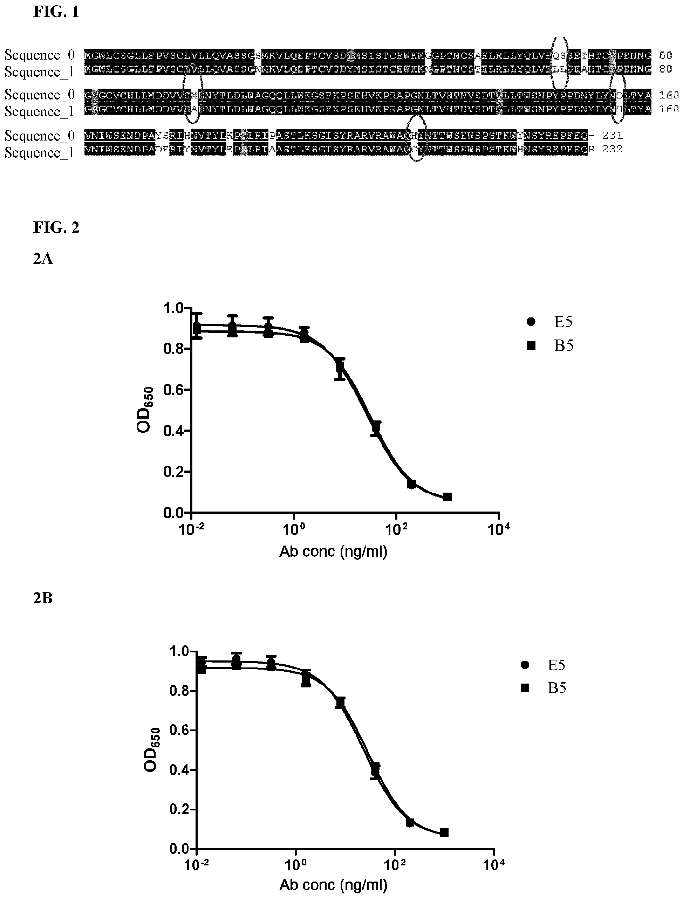

Sequences of the human IL-4Rα and cynomolgus IL-4Rα were aligned as shown in FIG. 1. It is speculated that, some positions where the sequence of the human IL-4Rα differs from that of the cynomolgus IL-4Rα are likely to be critical positions which are responsible for the binding of an antibody to IL-4Rα, and enable the antibody to bind to the human sequence, not to the cynomolgus sequence. Therefore the position(s) in the human IL-4Rα sequence, at which the amino acid(s) is(are) different from that(those) in the cynomolgus IL-4Rα sequence, were subjected to single point mutation or combined mutation, to change the amino acid(s) to the amino acid(s) at the corresponding position(s) in the cynomolgus IL-4Rα sequence; and the non-Alanine amino acid(s) in the vicinity of the position(s), at which differential amino acid(s) are present, were subjected to single point mutation or combined mutation, to change the amino acid(s) to Alanine. Then mutants of human sIL-4Rα having different mutation combinations were constructed (see Table 3 below) and their activity was verified following the procedure described in "(II) Cell functional activity detection method".

**Table 3. Mutants of human sIL-4Rα**

| No. | Mutation or mutation combination | No. | Mutation or mutation combination |
|---|---|---|---|
| sIL-4R-QSMDH | sIL-4R-L67Q/L68S/A96M/H1 56D/C207H | sIL-4R-63/64 | sIL-4R-Q63A/L64A |
| sIL-4R-MDH | sIL-4R-A96M/H156D/C207H | sIL-4R-65/66 | sIL-4R-V65A/F66A |
| sIL-4R-QSDH | sIL-4R-L67Q/L68S/H156D/C2 07H | sIL-4R-92 | sIL-4R-D92A |
| sIL-4R-QSMH | sIL-4R-L67Q/L68S/A96M/C2 07H | sIL-4R-94 | sIL-4R-V94A |
| sIL-4R-QSMD | sIL-4R-L67Q/L68S/A96M/H1 56D | sIL-4R-97 | sIL-4R-D97A |
| sIL-4R-S | sIL-4R-L68S | sIL-4R-92/94/97 | sIL-4R-D92A/V94A/ D97A |
| sIL-4R-Q | sIL-4R-L67Q | sIL-4R-92/94 | sIL-4R-D92A/V94A |
| | | sIL-4R-92/97 | sIL-4R-D92A/D97A |
| | | sIL-4R-94/97 | sIL-4R-V94A/D97A |

### 2.3 Binding activity detection

The binding activity of the 6 humanized high activity antibodies to antigens was detected following the procedure described in "(I) Binding activity detection method", by using the human sIL-4Rα protein and the different mutants of sIL-4Rα shown in Table 3 as the antigens and the antibodies as primary antibodies. The results are shown in Table 4.

The binding activity of the 9 chimeric antibodies to antigens was detected similarly, by using the human sIL-4Rα protein and the different mutants of sIL-4Rα shown in Table 3 as the antigens and the antibodies as primary antibodies. The results are shown in Table 5.

It was found through screening that the binding activity of the antibodies to the sIL-4Rα mutants with mutated amino acid(s) at those positions having different amino acids between human and cynomolgus sIL-4Rα and in the vicinity of the positions changed, or even reversed, compared to their binding activity to sIL-4Rα.

**Table 4. Binding of the antibodies to human sIL-4Rα or mutants thereof (EC50, ng/ml)**

| Antigen | B5 | E5 | B4 | E4 | B7 | E7 |
|---|---|---|---|---|---|---|
| sIL-4R-His | binding (18.4) | binding (12.2) | binding (10.8) | binding (13.2) | binding (9.47) | binding (12.1) |
| sIL-4R-QSMDH | weak binding (2.48e+03) | binding (646) | weak binding (2.29e+03) | weak binding (1.15e+04) | weak binding (1.43e+04) | weak binding (6.45e+03) |
| sIL-4R-MDH | binding (13.2) | binding (9.06) | binding (9.58) | binding (9.54) | binding (9.12) | binding (8.81) |
| sIL-4R-QSDH | binding (43.1) | binding (23.2) | binding (84.5) | binding (83.9) | binding (328) | binding (244) |
| sIL-4R-QSMH | binding (674) | binding (512) | weak binding (1.54e+03) | weak binding (1.67e+03) | weak binding (3.72e+03) | weak binding (2.01e+03) |
| sIL-4R-QSMD | binding (345) | binding (174) | weak binding (1.64e+03) | weak binding (2.39e+03) | weak binding (2.32e+03) | weak binding (5.26e+03) |
| sIL-4R-63/64 | binding (18.3) | binding (12.9) | binding (15.9) | binding (15.3) | binding (29.4) | binding (24.1) |
| sIL-4R-65/66 | binding (14.8) | binding (11.5) | binding (13.2) | binding (12.3) | binding (11.1) | binding (12.1) |
| sIL-4R-Q | binding (16.8) | binding (11) | binding (12.7) | binding (11.4) | binding (11.1) | binding (12.3) |
| sIL-4R-S | binding (19.3) | binding (10.9) | binding (16.8) | binding (15.6) | binding (35.5) | binding (35) |
| sIL-4R-92 | binding (24.6) | binding (14.1) | binding (31.3) | binding (29.2) | binding (166) | binding (131) |
| sIL-4R-94 | binding (19.9) | binding (14.4) | binding (18.9) | binding (15) | binding (12.9) | binding (15.6) |
| sIL-4R-97 | N.D. | N.D. | N.D. | N.D. | N.D. | N.D. |
| sIL-4R-92/94/97 | N.D. | N.D. | N.D. | N.D. | N.D. | N.D. |
| sIL-4R-92/94 | binding (19.4) | binding (11.8) | binding (25) | binding (23.5) | binding (162) | binding (126) |
| sIL-4R-92/97 | N.D. | N.D. | N.D. | N.D. | N.D. | N.D. |
| sIL-4R-94/97 | weak binding (2.33e+03) | N.D. | N.D. | N.D. | N.D. | N.D. |

| | | | | | | |
|---|---|---|---|---|---|---|
| "N.D." in the Table means no binding activity exhibited, even the antibody concentration reached 2500 ng/ml. | | | | | | |

As is clear from the data in Table 4, with respect to the five positions L67, L68, A96, H156, and C207, the amino acid residue at any one of them was mutated to the amino acid residue at the corresponding position in cynomolgus IL-4Rα; and when L67 and L68 were included in the combinations of the mutated positions, the binding activity of the humanized antibodies provided in the present disclosure was significantly lower compared with their binding activity to sIL-4R-His; meanwhile, when L67 and L68 were mutated individually, the humanized antibodies of the present disclosure maintained their original binding activity to sIL-4R-His.

With respect to the three positions D92, V94, and D97, the amino acid residue at any one of them was mutated to Alanine; and when D97 was included in the combinations of the mutated positions, or only D97 was mutated, the binding activity of the humanized antibodies provided in the present disclosure was significantly lower compared with their binding activity to sIL-4R-His.

**Table 5. Binding of the antibodies to cynomolgus sIL-4Rα, human sIL-4Rα or mutants thereof (EC50, ng/ml)**

| Antigen | Y0188-1Q | Y0188-2Q | Y0188-3Q | Y0188-4Q | Y0188-6Q | Y0188-8Q | Y0188-9Q | Y0188-10Q |
|---|---|---|---|---|---|---|---|---|
| sIL-4R-His | binding (9.67) | binding (10.2) | binding (25.8) | binding (12.3) | binding (10.6) | binding (11.4) | binding (12.7) | binding (13.7) |
| cyno sIL-4R-His | N.D. | N.D. | binding (445) | N.D. | binding (665) | N.D. | N.D. | binding (491) |
| sIL-4R-QSMDH | N.D. | binding (220) | N.D. | binding (34) | binding (8.57) | binding (293) | binding (413) | N.D. |
| sIL-4R-MDH | binding (8.55) | binding (10.2) | N.D. | binding (9.09) | binding (10.5) | binding (11.7) | binding (9.82) | binding (9.93) |
| sIL-4R-QSDH | binding (230) | binding (32.8) | binding (15.9) | binding (17.8) | binding (8.79) | binding (37) | binding (336) | N.D. |
| sIL-4R-QSMH | N.D. | binding (89.2) | N.D. | binding (25) | binding (13.7) | binding (74) | binding (130) | N.D. |
| sIL-4R-QSMD | N.D. | binding (39.5) | N.D. | binding (14.6) | binding (12.6) | binding (42.9) | binding (57.7) | N.D. |
| sIL-4R-63/64 | binding (6.52) | binding (8.08) | weak binding (1.36e+03) | N.D. | N.D. | binding (9.8) | weak binding (6.94e+03) | weak binding (4.02e+03) |
| sIL-4R-65/66 | binding (16.4) | binding (11.1) | binding (218) | binding (410) | binding (267) | binding (16.3) | binding (469) | binding (332) |
| sIL-4R-Q | binding (6.18) | binding (6.15) | binding (23.5) | binding (9.86) | binding (12.6) | binding (10.3) | binding (8.78) | binding (14.8) |
| sIL-4R-S | binding (5.89) | binding (6.68) | binding (22.9) | binding (4.79) | binding (7.61) | binding (11.4) | binding (14.2) | binding (132) |
| sIL-4R-92 | weak binding (1.1e+03) | binding (10.6) | N.D. | binding (31.3) | binding (105) | binding (9.57) | weak binding (1.49e+03) | binding (160) |
| sIL-4R-94 | binding (4.25) | binding (6.31) | binding (43.4) | binding (6.61) | binding (7.61) | binding (6.97) | binding (8.74) | binding (13.3) |
| sIL-4R-97 | N.D. | binding (8.05) | N.D. | N.D. | N.D. | binding (8.69) | N.D. | N.D. |
| sIL-4R-92/94/97 | N.D. | binding (15.3) | N.D. | N.D. | N.D. | binding (13.2) | N.D. | N.D. |
| sIL-4R-92/94 | binding (10.1) | binding (10.5) | N.D. | binding (13) | binding (17.4) | binding (6.98) | binding (27.6) | binding (25.8) |
| sIL-4R-92/97 | N.D. | binding (11.4) | N.D. | N.D. | N.D. | binding (6.99) | N.D. | N.D. |
| sIL-4R-94/97 | N.D. | binding (10.9) | N.D. | N.D. | N.D. | binding (7.06) | N.D. | N.D. |

| | | | | | | | | |
|---|---|---|---|---|---|---|---|---|
| "N.D." in the Table means no binding activity exhibited, even the antibody concentration reached 2500 ng/ml. | | | | | | | | |

As is clear from the data in Table 5, with respect to the three positions D92, V94, and D97, the amino acid residue at any one of them was mutated to Alanine; and when D97 was included in the combinations of the mutated positions, or only D97 was mutated, the binding activity of the chimeric antibodies provided in the present disclosure, except for Y0188-2Q and Y0188-8Q, was significantly lower compared with their binding activity to sIL-4R-His.

With respect to the five positions L67, L68, A96, H156, and C207, the amino acid residue at any one of them was mutated to the amino acid at the corresponding position in cynomolgus IL-4Rα; and when L67 and L68 or L67, L68, and A96 were included in the combinations of the mutated positions, the binding activity of the chimeric antibodies Y0188-1Q, Y0188-3Q, and Y0188-10Q was significantly lower compared with their binding activity to sIL-4R-His; meanwhile, when L67 and L68 were mutated individually, the chimeric antibodies, except for Y0188-2Q and Y0188-8Q, all maintained their original binding activity to sIL-4R-His. Furthermore, with respect to A96, when A96 was included in the combinations of the mutated positions, the binding activity of the chimeric antibody Y0188-3Q was significantly lower compared with its binding activity to sIL-4R-His.

With respect to the two positions Q63 and L64, when both positions were mutated to Alanine, the binding activity of the chimeric antibodies, except for Y0188-1Q, Y0188-2Q, and Y0188-8Q, was significantly lower compared with their binding activity to sIL-4R-His.

### EXAMPLE 3 Stability of the antibodies

The antibodies E5, and B5 were formulated in 10 mM Histidine, 150 mM NaCl, pH 6 at 0.59 mg/mL, 0.54 mg/mL, and 6.44 mg/mL, respectively, stored at 40 °C for 2 weeks and 4 weeks, and detected following the procedure described in "(II) Cell functional activity detection method". A graph of antibody concentration versus OD650 value was plotted (FIG. 2), from which IC50 (ng/mL) was then calculated. The results are shown in Table 6 below.

**Table 6. Stability results of the antibodies**

| Antibody | IC50 (ng/ml; 2 weeks) (blocking IL-4) | IC50 (ng/ml; 4 weeks) (blocking IL-4) |
|---|---|---|
| E5 | 26.7 | 24.4 |
| B5 | 31.1 | 29.1 |

The above description of the embodiments of the present invention is not intended to limit the present invention, and those skilled in the art may make various changes and modifications to the present invention without departing from the spirit of the present invention, which should fall within the scope of the appended claims.

## Claims

1. An antibody or antigen-binding fragment thereof that binds to human interleukin-4 receptor (IL-4R), the antibody or antigen-binding fragment thereof binding to an epitope which is located on the alpha chain of human IL-4R (IL-4Rα) and comprises one or more of amino acid residues D92, V94, D97, L67, L68, A96, H156, C207, Q63, and L64 in the amino acid sequence set forth in SEQ ID NO. 1.

2. The antibody or antigen-binding fragment thereof according to claim 1, wherein the epitope comprises one or more of amino acid residues D92, V94, and D97 in the amino acid sequence set forth in SEQ ID NO. 1; more preferably, the epitope comprises the following amino acid residues in the amino acid sequence set forth in SEQ ID NO. 1:
(1) D92;
(2) D97;
(3) D92, V94, and D97;
(4) D92 and V94;
(5) D92 and D97; or
(6) V94 and D97;
preferably, the antibody or antigen binding fragment thereof binds to an epitope of human IL-4Rα comprising the following amino acid residues in the amino acid sequence set forth in SEQ ID NO. 1: (i) Q63 and L64.

3. The antibody or antigen-binding fragment thereof according to claim 1 or 2, wherein the antibody or antigen binding fragment thereof has no a cross-species binding activity to cynomolgus IL-4R ; preferably, the antibody or antigen binding fragment thereof does not bind to cynomolgus IL-4R; more preferably, does not bind to cynomolgus IL-4Rα; and further preferably, does not bind to the amino acid sequence set forth in SEQ ID NO. 2;
preferably, the epitope comprises one or more of amino acid residues L67, L68, and A96 in the amino acid sequence set forth in SEQ ID NO. 1; preferably, the epitope comprises:
① L67 and L68;
② L67, L68, and A96; or
③ A96;
more preferably, the epitope comprises the following amino acid residues in the amino acid sequence set forth in SEQ ID NO. 1:
(A) L67, L68, A96, H156, and C207;
(B) A96, H156, and C207;
(C) L67, L68, H156, and C207;
(D) L67, L68, A96, and C207; or
(E) L67, L68, A96, and H156.

4. The antibody or antigen-binding fragment thereof according to any one of claims 1 to 3, wherein the epitope comprises the following amino acid residues in the amino acid sequence set forth in SEQ ID NO. 1:
(P-1) D97, L67, and L68;
(P-2) D97, L67, L68, A96, and D92;
(P-3) D97, L67, L68, and D92;
(P-4) D97, Q63, and L64;
(P-5) D97, Q63, L64, and D92;
(P-6) D97, Q63, L64, D92, and A96; or
(P-7) D97, Q63, L64, L67, and L68.

5. The antibody or antigen-binding fragment thereof according to any one of claims 1 to 4, wherein the antibody or antigen-binding fragment thereof comprises the following heavy chain complementarity determining regions HCDR1, HCDR2, HCDR3 and light chain complementarity determining regions LCDR1, LCDR2, LCDR3 in the heavy chain variable region and the ligh chain variable region:
(1) HCDR1, HCDR2, and HCDR3 set forth in SEQ ID NOs. 35, 36, and 37; and, LCDR1, LCDR2, and LCDR3 set forth in SEQ ID NOs. 53, 54, and 55;
(2) HCDR1, HCDR2, and HCDR3 set forth in SEQ ID NOs. 41, 42, and 43; and, LCDR1, LCDR2, and LCDR3 set forth in SEQ ID NOs. 59, 60, and 61;
(3) HCDR1, HCDR2, and HCDR3 set forth in SEQ ID NOs. 44, 45, and 46; and, LCDR1, LCDR2, and LCDR3 set forth in SEQ ID NOs. 56, 57, and 62;
(4) HCDR1, HCDR2, and HCDR3 set forth in SEQ ID NOs. 44, 47, and 46; and LCDR1, LCDR2, and LCDR3 set forth in SEQ ID NOs. 63, 64, and 65;
(5) HCDR1, HCDR2, and HCDR3 set forth in SEQ ID NOs. 48, 49, and 46; and, LCDR1, LCDR2, and LCDR3 set forth in SEQ ID NOs. 66, 67, and 68;
(6) HCDR1, HCDR2, and HCDR3 set forth in SEQ ID NOs. 44, 50, and 51; and, LCDR1, LCDR2, and LCDR3 set forth in SEQ ID NOs. 56, 57, and 69; or
(7) HCDR1, HCDR2, and HCDR3 set forth in SEQ ID NOs. 52, 36, and 37; and, LCDR1, LCDR2, and LCDR3 set forth in SEQ ID NOs. 53, 54, and 55;
preferably, the heavy chain variable region comprises a sequence as follows: the amino acid sequence set forth in SEQ ID NO. 3, SEQ ID NO. 7, SEQ ID NO. 9, SEQ ID NO. 11, SEQ ID NO. 15, SEQ ID NO. 17, SEQ ID NO. 19, SEQ ID NO. 23, or SEQ ID NO. 27 or an amino acid sequence having at least 75% identity to the amino acid sequence; and/or, preferably, the light chain variable region comprises a sequence as follows: the amino acid sequence set forth in SEQ ID NO. 4, SEQ ID NO. 8, SEQ ID NO. 10, SEQ ID NO. 12, SEQ ID NO. 16, SEQ ID NO. 18, SEQ ID NO. 20, SEQ ID NO. 28, SEQ ID NO. 33, or SEQ ID NO. 34 or an amino acid sequence having at least 75% identity to the amino acid sequence.

6. The antibody or antigen-binding fragment thereof according to any one of claims 1 to 5, wherein in the antibody or antigen-binding fragment thereof:
(1) the heavy chain variable region comprises the amino acid sequence set forth in SEQ ID NO. 3 or an amino acid sequence having at least 75% identity to the amino acid sequence set forth in SEQ ID NO. 3; and, the light chain variable region comprises the amino acid sequence set forth in SEQ ID NO. 4 or an amino acid sequence having at least 75% identity to the amino acid sequence set forth in SEQ ID NO. 4;
(2) the heavy chain variable region comprises the amino acid sequence set forth in SEQ ID NO. 7 or an amino acid sequence having at least 75% identity to the amino acid sequence set forth in SEQ ID NO. 7; and, the light chain variable region comprises the amino acid sequence set forth in SEQ ID NO. 8 or an amino acid sequence having at least 75% identity to the amino acid sequence set forth in SEQ ID NO. 8;
(3) the heavy chain variable region comprises the amino acid sequence set forth in SEQ ID NO. 9 or an amino acid sequence having at least 75% identity to the amino acid sequence set forth in SEQ ID NO. 9; and, the light chain variable region comprises the amino acid sequence set forth in SEQ ID NO. 10 or an amino acid sequence having at least 75% identity to the amino acid sequence set forth in SEQ ID NO. 10;
(4) the heavy chain variable region comprises the amino acid sequence set forth in SEQ ID NO. 11 or an amino acid sequence having at least 75% identity to the amino acid sequence set forth in SEQ ID NO. 11; and, the light chain variable region comprises the amino acid sequence set forth in SEQ ID NO. 12 or an amino acid sequence having at least 75% identity to the amino acid sequence set forth in SEQ ID NO. 12;
(5) the heavy chain variable region comprises the amino acid sequence set forth in SEQ ID NO. 15 or an amino acid sequence having at least 75% identity to the amino acid sequence set forth in SEQ ID NO. 15; and, the light chain variable region comprises the amino acid sequence set forth in SEQ ID NO. 16 or an amino acid sequence having at least 75% identity to the amino acid sequence set forth in SEQ ID NO. 16;
(6) the heavy chain variable region comprises the amino acid sequence set forth in SEQ ID NO. 17 or an amino acid sequence having at least 75% identity to the amino acid sequence set forth in SEQ ID NO. 17; and, the light chain variable region comprises the amino acid sequence set forth in SEQ ID NO. 18 or an amino acid sequence having at least 75% identity to the amino acid sequence set forth in SEQ ID NO. 18;
(7) the heavy chain variable region comprises the amino acid sequence set forth in SEQ ID NO. 19 or an amino acid sequence having at least 75% identity to the amino acid sequence set forth in SEQ ID NO. 19; and, the light chain variable region comprises the amino acid sequence set forth in SEQ ID NO. 20 or an amino acid sequence having at least 75% identity to the amino acid sequence set forth in SEQ ID NO. 20;
(8) the heavy chain variable region comprises the amino acid sequence set forth in SEQ ID NO. 27 or an amino acid sequence having at least 75% identity to the amino acid sequence set forth in SEQ ID NO. 27; and, the light chain variable region comprises the amino acid sequence set forth in SEQ ID NO. 34 or an amino acid sequence having at least 75% identity to the amino acid sequence set forth in SEQ ID NO. 34;
(9) the heavy chain variable region comprises the amino acid sequence set forth in SEQ ID NO. 27 or an amino acid sequence having at least 75% identity to the amino acid sequence set forth in SEQ ID NO. 27; and, the light chain variable region comprises the amino acid sequence set forth in SEQ ID NO. 33 or an amino acid sequence having at least 75% identity to the amino acid sequence set forth in SEQ ID NO. 33;
(10) the heavy chain variable region comprises the amino acid sequence set forth in SEQ ID NO. 27 or an amino acid sequence having at least 75% identity to the amino acid sequence set forth in SEQ ID NO. 27; and, the light chain variable region comprises the amino acid sequence set forth in SEQ ID NO. 28 or an amino acid sequence having at least 75% identity to the amino acid sequence set forth in SEQ ID NO. 28;
(11) the heavy chain variable region comprises the amino acid sequence set forth in SEQ ID NO. 23 or an amino acid sequence having at least 75% identity to the amino acid sequence set forth in SEQ ID NO. 23; and, the light chain variable region comprises the amino acid sequence set forth in SEQ ID NO. 34 or an amino acid sequence having at least 75% identity to the amino acid sequence set forth in SEQ ID NO. 34;
(12) the heavy chain variable region comprises the amino acid sequence set forth in SEQ ID NO. 23 or an amino acid sequence having at least 75% identity to the amino acid sequence set forth in SEQ ID NO. 23; and, the light chain variable region comprises the amino acid sequence set forth in SEQ ID NO. 33 or an amino acid sequence having at least 75% identity to the amino acid sequence set forth in SEQ ID NO. 33; or
(13) the heavy chain variable region comprises the amino acid sequence set forth in SEQ ID NO. 23 or an amino acid sequence having at least 75% identity to the amino acid sequence set forth in SEQ ID NO. 23; and, the light chain variable region comprises the amino acid sequence set forth in SEQ ID NO. 28 or an amino acid sequence having at least 75% identity to the amino acid sequence set forth in SEQ ID NO. 28.

7. The antibody or antigen-binding fragment thereof according to any one of claims 1 to 6, wherein the antibody or antigen-binding fragment thereof binds to human interleukin-4 receptor (IL-4R), preferably to the alpha chain of human IL-4R (IL-4Rα), more preferably to human soluble IL-4Rα (sIL-4Rα);
preferably, the antibody is a monoclonal antibody or a single chain antibody;
preferably, the antibody is a murine antibody, a chimeric antibody or a fully or partially humanized antibody; preferably, the antigen-binding fragment is a fragment of the antibody that is capable of specifically binding to IL-4R or IL-4Rα.

8. The antibody or antigen-binding fragment thereof according to any one of claims 1 to 7, wherein the antibody or antigen-binding fragment thereof further comprises a human or murine constant region, preferably a human or murine heavy chain constant region (CH) and/or a light chain constant region (CL);
preferably, the antibody or antigen-binding fragment thereof comprises a heavy chain and a light chain;
preferably, the antibody is an immunoglobulin, in particular IgA, IgD, IgE, IgG, or IgM, for example a human IgA, IgD, IgE, IgG, or IgM, more preferably a human IgG1, IgG2, IgG3, or IgG4 subtype;
more preferably, the antibody or antigen-binding fragment thereof comprises a heavy chain constant region of an IgG, IgA, IgM, IgD, or IgE and/or a light chain constant region of a kappa or lambda type; for example, the heavy chain constant region is of an IgG (e.g., IgG1 or IgG4) type and the light chain constant region is of a kappa type.

9. A nucleic acid molecule comprising a nucleotide sequence encoding a heavy chain variable region, a light chain variable region, a heavy chain, and/or a light chain of the antibody or antigen-binding fragment thereof according to any one of claims 1 to 8.

10. A vector comprising the nucleic acid molecule according to claim 9.

11. A host cell comprising the nucleic acid molecule according to claim 9 or the vector according to claim 10, or transformed or transfected with the nucleic acid molecule according to claim 9 or the vector according to claim 10.

12. A composition comprising the antibody or antigen-binding fragment thereof according to any one of claims 1 to 8, the nucleic acid molecule according to claim 9, the vector according to claim 10, or the host cell according to claim 11.

13. The composition according to claim 12, wherein the composition is a pharmaceutical composition; preferably, the pharmaceutical composition comprises a pharmaceutically acceptable excipient;
preferably, the pharmaceutical composition is prepared into dosage forms for oral administration and parenteral administration.

14. Use of the antibody or antigen-binding fragment thereof according to any one of claims 1 to 8, the nucleic acid molecule according to claim 9, the vector according to claim 10, the host cell according to claim 11, or the composition according to claim 12 or 13 in the manufacture of a medicament for the prevention, treatment or amelioration of a disease or disorder associated with human interleukin 4 receptor (IL-4R) or a disease or disorder associated with human interleukin 4 (IL-4) or human interleukin 13 (IL-13) signaling pathway.

15. A method for preventing, treating, or ameliorating a disease or disorder, the method comprising administering (e.g., a therapeutically effective amount of) the antibody or antigen-binding fragment thereof according to any one of claims 1 to 8, the nucleic acid molecule according to claim 9, the vector according to claim 10, the host cell according to claim 11, or the composition according to claim 12 or 13 to a subject in need thereof, wherein the disease or disorder is associated with human interleukin 4 receptor (IL-4R) or associated with human interleukin 4 (IL-4) or human interleukin 13 (IL-13) signaling pathway.

16. The use according to claim 14 or the method according to claim 15, wherein the disease or disorder includes an autoimmune disease, an allergic disease, a tumor, or a cancer;
preferably, the disease or disorder includes an autoimmune disease or an allergic disease, for example dermatitis (e.g. atopic dermatitis), asthma (e.g. inflammatory asthma, such as inflammatory asthma type 2), chronic esophagitis (e.g. eosinophilic esophagitis), eczema, rhinitis (e.g. allergic rhinitis), nasal polyps (e.g. chronic rhinosinusitis with nasal polyps), conjunctivitis, inflammatory bowel disease (e.g. ulcerative colitis, and crohn's disease), inflammatory neuropathy, arthritis (e.g. rheumatoid arthritis), multiple sclerosis, lupus erythematosus, psoriasis, or insulin and non-insulin dependent diabetes mellitus, and other immune or allergy related diseases;
alternatively, preferably, the disease or disorder includes a tumor or cancer, such as intestinal cancer, melanoma, skin cancer, breast cancer, uterine cancer, cervical cancer, endometrial cancer, ovarian cancer, testicular cancer, mesothelioma, prostate cancer, bladder cancer, anal cancer, glioblastoma, astrocytoma, liver cancer, kidney cancer, esophageal cancer, gastric cancer, lung cancer, head and neck cancer, myeloma, bone cancer, aids-related kaposi's sarcoma, hodgkin's or non-hodgkin's lymphoma, prostate cancer, prostate tumor, lymphoma, and pancreatic cancer.

17. The use or the method according to any one of claims 14 to 16, wherein the medicament is for the prevention, treatment or amelioration of one or more of the diseases or discorders as defined in claim 16;
preferably, the subject is a mammal, preferably a primate; and more preferably, the subject is a human.

18. A method for screening for a binding agent that specifically binds to human interleukin 4 receptor (IL-4R) or a blocking agent that specifically blocks human interleukin 4 (IL-4) or human interleukin 13 (IL-13) signaling pathway, the method comprising the steps as follows: contacting an agent to be screened with human IL-4R or part thereof, and then detecting whether the agent is capable of binding to the epitope as defined in any one of claims 1 to 3.

19. A method for evaluating efficacy of an agent, the method comprising the steps as follows: contacting the agent to be evaluated with human interleukin 4 receptor (IL-4R) or part thereof, and then detecting whether the agent is capable of binding to the epitope as defined in any one of claims 1 to 3.

20. The method for screening for a binding agent or a blocking agent according to claim 18 or the method for evaluating efficacy of an agent according to claim 19, wherein the agent to be screened or evaluated is an antibody, such as a polyclonal antibody or a monoclonal antibody; preferably, the antibody is obtained through immunizing an animal with human IL-4R or part thereof as an immunogen;
preferably, the detecting whether the agent is capable of binding to the epitope is performed by mutating one or more amino acid residues comprised by the epitope through site-directed mutagenesis of human IL-4R or part thereof, comparing the binding activity or affinity of the agent to the human IL-4R or part thereof after the mutation with that before the mutation, and identifying the agent capable of binding to the epitope, if the binding activity or affinity of the agent to the mutated human IL-4R or part thereof is significantly lower or even lost.

21. The method for screening for a binding agent or a blocking agent or the method for evaluating efficacy of an agent according to any one of claims 18 to 20, wherein the method includes the steps as follows:
1) mutating one or more amino acid residues comprised by the epitope through site-directed mutagenesis of human IL-4R or part thereof, to obtain a mutant of the human IL-4R or part thereof;
2) contacting an agent to screened or an agent to be evaluated with the human IL-4R or part thereof and the mutant of the human IL-4R or part thereof respectively, and detecting the binding activity or affinity thereto respectively;
3) identifying the agent capable of binding to the epitope, if the binding activity or affinity of the agent to the mutated human IL-4R or part thereof is significantly lower or even lost compared with the binding activity or affinity of the agent to the human IL-4R or part thereof.

22. The method for screening for a binding agent or a blocking agent or the method for evaluating efficacy of an agent according to any one of claims 18 to 21, wherein in step 1), one or more amino acid residues comprised by the epitope are mutated to Alanine or to an amino acid residue of the cynomolgus interleukin 4 receptor at the corresponding position, to obtain a mutant of human IL-4R or part thereof;
preferably, the part of human IL-4R is the alpha chain of human IL-4R, further human sIL-4Rα; preferably, the method is performed using human IL-4Rα and a mutant of human IL-4Rα is obtained;
preferably, the method is performed using human sIL-4Rα and one or more of the mutants of human sIL-4Rα listed in Table 3 in the description are used as the mutant in step 1).
